# EUROPEAN PATENT APPLICATION

(11) **EP 4 140 473 A1**
(43) Date of publication of application: **01.03.2023**
(21) Application number: 21020429.3
(22) Date of filing: 27.08.2021
(51) Int. Cl.: A61K 8/73, A61K 8/64, A61Q 19/08

(54) **COSMETIC COMPOSITIONS**

(71) Applicant: The Boots Company plc, Nottingham NG90 1BS (GB)
(72) Inventor: O'CONNOR, Clare, West Hallam Derbyshire DE7 6HG (GB); BELL, Michael David, Higham, Alfreton Derbyshire DE55 6EL (GB); NEWTON, Victoria Lynne, Beeston, Nottingham Nottinghamshire NG9 1GQ (GB)
(74) Representative: Teasdale, Andrew James

(57) **Abstract**

Cosmetic compositions, kits comprising cosmetic compositions, and methods and uses thereof are provided, wherein the cosmetic compositions comprise a tetrapeptide capable of stimulating protein synthesis, skin repair and regeneration in combination with oligo-alpha glucans.

## Description

### Field of the Invention

The invention relates to the field of skin repair. More particularly, the invention pertains to compositions, methods and kits, and uses thereof for enhancing the effect of skin repair agents.

### Background of the Invention

There is a focus within the cosmetics industry on providing ever more potent formulations to support the natural processes of skin repair.

The largest component of normal skin is the dermal extracellular matrix (ECM) which is a gel-like matrix produced by the cells that it surrounds. The ECM comprises two major elements, structural proteins and proteoglycans. Changes to skin come about naturally over time (ageing) as a result of changes in the cosmetic composition and crosslinked state of the elements of the ECM which cause changes at the structural and behavioural level. Structural changes affect the integrity of the skin. Ageing skin exhibits both an increased degradation of dermal extracellular matrix (ECM) proteins (such as collagen, laminin, elastin, fibronectin, fibrillin) and reduced production of the same proteins.

Cellular behavioural changes influence the rate of cell differentiation and proliferation, and therefore the rate of skin repair and renewal. The ECM is also believed to be responsible for producing cell signals which facilitate epithelial cell proliferation and migration, further influencing the ability of the skin to repair. Changes in the ECM also affect cell-adhesion, signalling, and cell-behaviour mediation.

Particularly important ECM proteins include collagen, fibrillin, fibronection and decorin. Collagen is responsible for providing elements of the structural integrity of the skin. Collagen in the dermal matrix is composed primarily of type I (80-85%) and type III (8-11%) collagens, both of which are fibrillar, or rod-shaped, collagens. The tensile strength of skin is predominately produced through the fibrillar cross-linked arrangement of the molecules of collagen I. There are also other functionally important although less abundant collagen types in skin, including collagen IV located at the basement membrane of the dermal epidermal junction. This forms sheet-like structures and is more pliable than the fibrillar collagens. Damage to the collagen network of the skin by, for example, free-radical damage, induces the generation of collagen fragments in the ECM, followed by skin regeneration and repair.

Fibrillin is a component of elastic fibres in the ECM and also contributes to the structural integrity of the skin. The most abundant fibrillin in elastic fibres of skin is the FBN1 encoded protein, fibrillin 1.

Fibronectin is an important glycoprotein of the ECM and is primarily responsible for mediating a wide variety of cellular interactions within the dermal ECM, playing an important role in cell adhesion, migration, growth and differentiation.

Decorin (DCN) is a proteoglycan, belonging to the small leucine-rich proteoglycan (SLRP) family. The peptide is believed to be important as a component of connective tissue and binds to type I collagen, regulating collagen ECM assembly.

Ageing changes in the skin can occur intrinsically as a consequence of time and extrinsically as a consequence of external mechanisms, including UV-related damage and pollution. Extrinsic mechanisms can result in the increased availability of reactive oxygen species (UV-ROS). These reactive species are known to cause ECM fragmentation and the upregulation of Matrix Metalloproteinases (MMPs) and other ECM proteases. ECM fragmentation may bring about damage to and/or remodelling of the structural ECM proteins and reduce the integrity of the skin.

The fragmentation of ECM proteins can also result in the release of small bioactive peptides or matrikines which act as cell signalling molecules promoting skin repair by upregulating protein production, cell proliferation and differentiation. Both intrinsic and extrinsic ageing can lead to protein fragmentation and the subsequent release of bioactive peptide matrikines.

A number of natural and synthetic peptide matrikines have been used in cosmetic compositions and products for a number of years to stimulate protein synthesis, skin repair and regeneration. The most commonly used peptides are Pal-KKTKS and the combination of palmitoyl oligopeptide (Pal-GHK), which acts as a messenger peptide for collagen renewal and consists of a sequence derived from collagen I, and palmitoyl tetrapeptide-7 (Pal-GQPR), which reduces the production of interleukin-6 (IL-6), therefore acting as anti-inflammatory and inhibiting ECM degradation, with sequence derived from immunoglobulin G. The said peptide combinations are available from Sederma under the trade names Matrixyl and Matrixyl 3000.

Circadian rhythm, also called the "body's clock" is a natural cycle of physical and behavioural changes that the body goes through over a roughly 24-hour period in response to environmental cues.

Skin cells have a natural circadian rhythm, which when synchronised correctly will have different phases of protection and repair. The protection phase is during a time that external factors (e.g. high UV exposure during the day) are more likely to damage existing genetic material and tissue and therefore cells will primarily be focused on preventing this damage of existing material rather than generation of new material. The repair phase occurs when the risk of damage to new material is at its lowest (e.g. during the night) and might consist of generation of new proteins to be utilised by the skin to lay down new extracellular matrix for example.

External stressors such as UV, oxidative stress, pollution and internal stress will disrupt the circadian rhythm of skin cells. The impact of this is twofold. Firstly, when out of synchronisation the cells will not be as responsive to the repair signals produced by the body in a protection phase and therefore the production of new material will be suboptimal. Secondly, the new material will be vulnerable to breakdown once produced as the environment is not correctly synchronised for optimal repair and not likely to be incorporated in new matrix optimally. Reestablishing these natural circadian rhythms would therefore be beneficial.

FR2989276A1 discloses the use of *Lindera Strychnifolia* extract to synchronise the circadian rhythm of the treated skin cells by restoring the expression of the CLOCK gene in the treated cells, resulting in skin with improved cutaneous parameters such as rate of hydration and radiance (assessed visually). Application of *Lindera Strychnifolia* extract was therefore shown to limit the signs of aging. It is noted in FR2989276A1 that the presence of oligo-alpha-glucans in the extract is essential to provide the effect.

### Summary of the Invention

It has been recognised by the inventors that application to the skin of peptides that stimulate protein synthesis, skin repair and regeneration does not produce reliable results, even under controlled conditions.

The inventors have surprisingly found that when a tetrapeptide that stimulates protein synthesis, skin repair and regeneration is used in combination with oligo-alpha-glucans capable of resetting the circadian rhythm of the skin cells, both the efficacy and the reliability of the tetrapeptide is increased relative to the tetrapeptide used in isolation. The oligo-alpha-glucans and tetrapeptide can be provided in one cosmetic composition, or in first and second cosmetic compositions as part of a kit.

Without wishing to be bound by theory, it is thought that there are at least two reasons that the invention provides this synergistic result. The first is that the skin cells, having been synchronised to the repair phase, are able to make ready use of the skin repair agent made available, before the skin repair agent is metabolised by the body or removed from the skin by washing. The second is that the skin cells are all in the repair phase at the same time, meaning that they are able to work concurrently, drawing the same resources from the body at the same time to make use of the skin repair agent.

According to a first aspect of the invention, there is provided a cosmetic composition comprising oligo-alpha-glucans and a tetrapeptide wherein the tetrapeptide is selected from the group consisting of:
a) tetrapeptides having the amino acid sequence U-LSXX-Z (SEQ ID No. 1) wherein L is used to denote amino acid Leucine and S is used to denote Serine, as per the internationally recognised single letter code for amino acids and X denotes an amino acid selected from the group consisting of Valine (V), Aspartic acid (D), Proline (P), Glycine (G) and mixtures thereof;
b) tetrapeptides having the amino acid sequence U-GPXG-Z (SEQ ID No. 2) wherein G is used to denote amino acid glycine and P denotes the amino acid proline, as per the internationally recognised single letter code for amino acids, X denotes an amino acid independently selected from the group consisting of Lysine (K), Glutamic acid (E), Serine (S) and mixtures thereof;
c) tetrapeptides having the amino acid sequence U-XXGD-Z (SEQ ID No. 3) wherein G is used to denote amino acid Glycine and D is used to denote amino acid Aspartic acid, as per the internationally recognised single letter code for amino acids and X denotes an amino acid selected from the group consisting of Glutamic acid (E), Lysine (K), Leucine (L), Alanine (A), Isoleucine (I), Arginine (R) and mixtures thereof;
d) tetrapeptides having the amino acid sequence U-QTAV-Z (SEQ ID No. 4) wherein Q is used to denote amino acid Glutamine, T is used to denote amino acid Threonine, A is used to denote amino acid Alanine and V is used to denote amino acid Valine, as per the internationally recognised single letter code for amino acids; and
e) combinations thereof,
wherein at the N-terminal end of the tetrapeptide, U is selected from the group consisting of H, -CO-R¹, -SO₂-R¹ and a biotinyl group, wherein at the C-terminal end of the tetrapeptide, Z is selected from the group consisting of OH, O R¹, NHR¹ and NR¹R², and wherein R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N.

According to a second aspect of the invention there is provided a cosmetic composition comprising oligo-alpha-glucans provided in the form of an extract from the roots of lindera strychnifolia, wherein, optionally the extract is subjected to an enzymatic hydrolysis step; and a tetrapeptide, wherein the tetrapeptide is selected from the group consisting of RSRK and U-GQPR peptides, wherein R is used to denote amino acid Arginine, S is used to denote amino acid Serine, K is used to denote amino acid Lysine, G is used to denote amino acid Glycine, Q is used to denote amino acid Glutamine, and P is used to denote the amino acid proline as per the internationally recognised single letter code for amino acids, wherein at the N-terminal end of the one or more tetrapeptide, U is selected from the group consisting of H, -CO-R¹, -SO₂-R¹ or a biotinyl group, and wherein R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N.

In some embodiments of the second aspect, the tetrapeptide of the cosmetic composition is N-palmitoyl-GQPR.

According to a third aspect of the invention there is provided a method for stimulating the production of dermal extracellular proteins in humans, the method comprising applying to the skin a cosmetic composition of the first or second aspect

According to a fourth aspect of the invention, there is provided a kit comprising a first cosmetic composition comprising oligo-alpha-glucans and a second cosmetic composition comprising a tetrapeptide, wherein the tetrapeptide is selected from the group consisting of:
a) tetrapeptides having the amino acid sequence U-LSXX-Z (SEQ ID No. 1) wherein L is used to denote amino acid Leucine and S is used to denote Serine, as per the internationally recognised single letter code for amino acids and X denotes an amino acid selected from the group consisting of Valine (V), Aspartic acid (D), Proline (P), Glycine (G) and mixtures thereof;
b) tetrapeptides having the amino acid sequence U-GPXG-Z (SEQ ID No. 2) wherein G is used to denote amino acid glycine and P denotes the amino acid proline, as per the internationally recognised single letter code for amino acids, X denotes an amino acid independently selected from the group consisting of Lysine (K), Glutamic acid (E), Serine (S) and mixtures thereof;
c) tetrapeptides having the amino acid sequence U-XXGD-Z (SEQ ID No. 3) wherein G is used to denote amino acid Glycine and D is used to denote amino acid Aspartic acid, as per the internationally recognised single letter code for amino acids and X denotes an amino acid selected from the group consisting of Glutamic acid (E), Lysine (K), Leucine (L), Alanine (A), Isoleucine (I), Arginine (R) and mixtures thereof;
d) tetrapeptides having the amino acid sequence U-QTAV-Z (SEQ ID No. 4) wherein Q is used to denote amino acid Glutamine, T is used to denote amino acid Threonine, A is used to denote amino acid Alanine and V is used to denote amino acid Valine;
e) tetrapeptides having the amino acid sequence RSRK (SEQ ID No. 51) and U-GQPR-Z (SEQ ID No. 52), most preferably N-palmitoyl-GQPR-OH, wherein R is used to denote amino acid Arginine, S is used to denote amino acid Serine, K is used to denote amino acid Lysine, G is used to denote amino acid Glycine, Q is used to denote amino acid Glutamine, and P is used to denote the amino acid proline as per the internationally recognised single letter code for amino acids; and
f) combinations thereof,
wherein at the N-terminal end of the tetrapeptide, U is selected from the group consisting of H, -CO-R¹, -SO₂-R¹ and a biotinyl group, wherein at the C-terminal end of the tetrapeptide, Z is selected from the group consisting of OH, O R¹, NHR¹ and NR¹R², and wherein R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N.

According to a fifth aspect of the invention there is provided a method for stimulating the production of dermal extracellular proteins in humans, the method comprising applying to the skin a first cosmetic composition comprising oligo-alpha-glucans according to the kit of the fourth aspect, and a second cosmetic composition comprising a tetrapeptide according to the kit of the fourth aspect.

In one embodiment of the method of the fifth aspect, the first cosmetic composition is applied before the second cosmetic composition.

According to a sixth aspect of the invention there is provided a use of a cosmetic composition of the first or second aspect, or the kit of the fourth aspect for the non-therapeutic cosmetic treatment to improve the condition of the skin and/or lines and/or wrinkles and/or imperfections.

### Detailed Description of the Invention

Unless otherwise indicated, a reference to cosmetic compositions or cosmetic compositions of the invention is a reference to the cosmetic compositions of all aspects of the invention, including the cosmetic compositions of the first and second aspect, and the first and second compositions of the kit of the fourth aspect.

### Oligo-Alpha-Glucans

The cosmetic compositions of the invention comprise oligo-alpha-glucans. Specific embodiments of the oligo-alpha-glucans are now described.

Oligo-alpha-glucans consist of two or more repeating units of glucose linked by alpha glycosidic bonds. These bonds can be alpha 1-2, alpha 1-3, alpha 1-4, and/or alpha 1-6 bonds.

Oligo-alpha-glucans are known to provide benefits to the skin, enhancing skin repair and moisture retention which in turn lead to improved skin appearance and health. The ability of oligo-alpha-glucans to synchronise the circadian rhythm of the cells has also been noted.

Without wishing to be bound by theory, it is thought the impact on the circadian rhythm arises from a mechanism equivalent to the serum shock. As detailed in Balsalobre et al, 1998, a dose of serum could induce the rhythmic expression of Perl, Per2, Reverb-α, Dbp, and Tef in cultured rat fibroblasts. The serum contained, amongst other things, glucose.

Oligo-alpha-glucans are the predominant form in which energy is both absorbed (from α-amylase acting on the starch we eat) and stored in the body (as glycogen). Their administration to the skin could therefore be producing a similar shock effect that synchronises the circadian rhythm of the skin cells.

The oligo-alpha-glucans of the invention may be botanically derived or they may be synthetically produced. In some preferred embodiments, the oligo-alpha-glucans are botanically derived, i.e., extracted from the roots, leaves, stems, flowers, and/or other parts of a plant. The oligo-alpha-glucans could be present as part of the whole plant extract, or as part of a particular fraction of the plant extract. For example, oligo-alpha-glucan containing extracts may be obtained from the stems, roots, leaves, seeds or fruit of *Avena sativa, Triticum aestivum, Brassica napus, Helianthus annuus, Prunus persica, Malus domestica, Pastinaca sativa, Daucus carota, Curcuma longa, Zingiber officinale, Lindera strychnifolia.*

In some preferred embodiments, the oligo-alpha-glucans are provided in the form of an extract from plants in the *Lindera* genus. In a preferred embodiment the oligo-alpha-glucans are provided in the form of an extract from *Lindera strychnifolia,* also known as *Lindera aggregata* or evergreen lindera. In a most preferred embodiment, the oligo-alpha-glucans are provided in the form of an extract from the roots of *Lindera strychnifolia.* The cosmetic composition of the second aspect comprises oligo-alpha-glucans provided in the form of an extract from the roots of lindera strychnifolia.

In some embodiments, the extract of *Lindera strychnifolia,* which comprises oligo-alpha-glucans, is used in the invention. Alongside oligo-alpha-glucans, the extract of *Lindera strychnifolia* comprises linderol (borneol), linderane, linderasure, lindesterene, linderene acetate, isolinderoxide, linderoxide, sesquiterpene lactones, bisesquiterpene, strychnilactone, strychnistenolide and its acetate and alkaloids and other sesquiterpenes such as isolinderalactone, neolinderalactone, lindestrenolide lindenone, linderazulene, chamazulene, laurolitsine, isogermafurene.

In some embodiments, the plant extract or fraction of the plant extract containing the oligo-alpha-glucans is hydrolysed. The hydrolysis process breaks down the polysaccharides in the plant extract or fraction of the plant extract to provide shorter chain glucans. The method of hydrolysis may be acid-base hydrolysis or enzymatic hydrolysis with glycoside hydrolases. In a preferred embodiment the method of hydrolysis is enzymatic hydrolysis.

In some embodiments, the oligo-alpha-glucans are provided in the form of an extract from the roots of lindera strychnifolia, wherein the extract is subjected to an enzymatic hydrolysis step.

In some embodiments, the oligo-alpha-glucans are prepared by the steps of extracting the powdered root of *Lindera strychnifolia* in water, exposing the extract to enzymatic hydrolysis and separating the soluble fraction containing the desired oligo-alpha-glucans from the insoluble fractions of the hydrolysate by methods known in the art. The extract can be further purified to enhance the oligo-alpha-glucan content and remove impurities.

The cosmetic composition of the invention may comprise the oligo-alpha-glucans in an amount effective to synchronise the circadian rhythm of the skin cells through regulation of CLOCK gene. For example, the oligo-alpha-glucans may be present in the cosmetic composition in an amount from about 0.00001 % to about 5 % by weight of the total composition, e.g. from about 0.00005 % to about 5 % by weight of the total composition, from about 0.0001 % to about 5 % by weight of the total composition from about 0.0001 % to about 4 % by weight of the total composition, from about 0.0001 % to about 3 % by weight of the total composition, from about 0.0001 % to about 2 % by weight of the total composition, from about 0.0001 % to about 1.5 % by weight of the total composition, from about 0.0001 % to about 1 % by weight of the total composition. In one embodiment, the oligo-alpha-glucans are present in an amount of from about 0.0001 % to about 0.5 % by weight of the total composition. Where the cosmetic composition forms part of a kit, as in the fourth aspect, the total cosmetic composition refers to the cosmetic composition comprising the oligo-alpha-glucans.

In some embodiments, the oligo-alpha-glucans consist of only one type of oligo-alpha-glucan with a single molecular weight and a single isomer of this molecular weight. In other embodiments the oligo-alpha-glucan comprises multiple types of oligo-alpha-glucans with varying degrees of polymerisation and multiple isomers of each molecular weight.

The oligo-alpha-glucans of the invention can have a linear or a branched structure or can be a combination of oligo-alpha-glucans with linear or branched structures.

The degree of polymerization is the number of monomeric units in a macromolecule or polymer or oligomer molecule. Herein, the degree of polymerisation is the number average, that is, the number average molecular weight of the oligo-alpha-glucans divided by the molecular weight of the monomer. In some embodiments, the oligo-alpha-glucan has a degree of polymerisation greater than or equal to 2 and less than or equal to 50, e.g., greater than or equal to 2 and less than or equal to 40 greater than or equal to 2 and less than or equal to 20, greater than or equal to 2 and less than or equal to 10. In a preferred embodiment, the oligo-alpha-glucan has a degree of polymerisation of from 2 to 6.

In some embodiments at least 50 % of the oligo-alpha-glucans will consist of from 2 to 6 glucose monomers linked by glycosidic bonds. For example, at least 55%, at least 60 %, at least 70 %, at least 80 %, at least 90 %, at least 95 % or at least 99 % of the oligo-alpha-glucans will consist of from 2 to 6 glucose monomers. In a preferred embodiment, at least 80% of the oligo-alpha-glucans oligomers consist of 2 to 6 glucose monomers.

The degree of polymerisation or the cosmetic composition of the oligo-alpha-glucans can be determined using High Pressure Liquid Chromatography (HPLC) in combination with mass spectrometry. In one embodiment, the mass spectrometry can be matrix assisted laser desorption/ionisation time-off-flight (MALDI-TOF) mass spectrometry. In one embodiment the method of determination is according to the operating conditions described in Hammad et al., J. Am. Soc. Mass Spectrom, 2009, 20, 1224-1234. ASTM D5896-96(2019)e1 can also be referenced.

The oligo-alpha-glucans of the invention may comprise at least 25% alpha 1-2 glycosidic bonds, for example, at least 35%, at least 50%, at least 75% or at least 90% alpha 1-2 glycosidic bonds. The oligo-alpha-glucans of the invention may comprise at least 25% alpha 1-3 glycosidic bonds, for example, at least 35%, at least 50%, at least 75% or at least 90% alpha 1-3 glycosidic bonds. The oligo-alpha-glucans of the invention may comprise at least 25% alpha 1-4 glycosidic bonds, for example, at least 35%, at least 50%, at least 75% or at least 90% alpha 1-4 glycosidic bonds. The oligo-alpha-glucans of the invention may comprise at least 25% alpha 1-6 glycosidic bonds, for example, at least 35%, at least 50%, at least 75% or at least 90% alpha 1-6 glycosidic bonds.

In some embodiments, the oligo-alpha-glucan comprises branched oligo-alpha-glucans with alpha 1-4 glycosidic bonds in the backbone and alpha 1-6 linkages to branched units and/or oligo-alpha-glucans with alpha 1-6 glycosidic bonds in the backbone and alpha 1-2 linkages to branched units. In some embodiments the oligo-alpha-glucans may comprise linear oligo-alpha-glucans with alpha 1-4 glycosidic bonds in the linear polymer, with alpha 1-6 glycosidic bonds in the linear polymer, with alpha 1-3 glycosidic bonds in the linear polymer and/or with alpha 1-2 glycosidic bonds in the linear polymer. In some embodiments where the oligo-alpha-glucan is linear, the oligo-alpha-glucans may comprise only one type of glycosidic bond.

### Tetrapeptides

The tetrapeptides of all aspects of the invention are provided to stimulate protein synthesis, skin repair and regeneration. It has been found that the provision to the skin of these tetrapeptides with oligo-alpha-glucans shows enhanced activity over either component alone.

The cosmetic compositions and kits of the invention may comprise the tetrapeptide in an amount effective to stimulate protein synthesis, skin repair and regeneration in the skin. For example, the tetrapeptide may be present in an amount from about 0.00001 % to about 5 % by weight of the total composition, e.g. from about 0.00001 % to about 3 % by weight of the total composition, from about 0.0001 % to about 1 % by weight of the total composition, from about 0.0005 % to about 0.5 % by weight of the total composition. In a preferred embodiment, the tetrapeptide is present in an amount of 0.0005 % to 0.1 % by weight of the total cosmetic composition. Where the cosmetic composition forms part of a kit, as in the fourth aspect, the total cosmetic composition refers to the cosmetic composition comprising the tetrapeptide.

In some embodiments of the cosmetic compositions and kits of the invention, the tetrapeptide is present in an amount of 1:2 to 40:1 parts tetrapeptide to oligo-alpha-glucans. For example, from 1:2 to 30:1, from 1:2 to 20:1, from 1:2 to 10:1, from 1:1 to 10:1, or from 1:1 to 5:1 parts tetrapeptide to oligo-alpha-glucans. In a preferred embodiment, the tetrapeptide is present in an amount of from 1:2 to 20:1 parts tetrapeptide to oligo-alpha-glucans.

In some embodiments of the cosmetic compositions and kits of the invention the tetrapeptide may be provided in a controlled release form. For example, the tetrapeptide may be encapsulated in vesicles, such as liposomes, or in an inorganic matrix. By controlled release it is meant that the release rate at which the tetrapeptides become available to the skin following application of the cosmetic composition is delayed or slowed relative to an immediate release form (e.g. where no encapsulation is used).

In some embodiments where a controlled release form is used, release rate is such the tetrapeptides are released over a period at least 2 hours following application to the skin, for example, a period of at least 4 hours, at least 6 hours, or at least 8 hours. In some embodiments, the release rate is such that not more than 80 % of the total amount of the tetrapeptides is released within 2 hours, for example, not more than 60 %, not more than 40% or not more than 20% of the tetrapeptides is released within 2 hours.

Examples of controlled release for topical cosmetic applications can be found in WO200862429A2 and EP2667844B1.

### Tetrapeptide of the First, Third, Fourth, Fifth and Sixth Aspects

The invention of the first, third, fourth, fifth and sixth aspects makes use of a tetrapeptide, specific embodiments of which are now described.

In one embodiment the tetrapeptide is selected from the group consisting of U-LSVD-Z (SEQ ID No. 5), U-LSVP-Z (SEQ ID No. 6), U-LSVG-Z (SEQ ID No. 7), U-LSDV-Z (SEQ ID No. 8), U-LSDP-Z (SEQ ID No. 9), U-LSDG-Z (SEQ ID No. 10), U-LSPV-Z (SEQ ID No. 11), U-LSPD-Z (SEQ ID No. 12), U-LSPG-Z (SEQ ID No. 13), U-LSGV-Z (SEQ ID No. 14), U-LSGD-Z (SEQ ID No. 15), U-LSGP-Z (SEQ ID No. 16), U-GPKG-Z (SEQ ID No. 17), U-GPEG-Z (SEQ ID No. 18), U-GPSG-Z (SEQ ID No. 19), U-GPPG-Z (SEQ ID No. 20), U-EKGD-Z (SEQ ID No. 21), U-ELGD-Z (SEQ ID No. 22), U-EAGD-Z (SEQ ID No. 23), U-EIGD-Z (SEQ ID No. 24), U-ERGD-Z (SEQ ID No. 25), U-KEGD-Z (SEQ ID No. 26), U-KLGD-Z (SEQ ID No. 27), U-KAGD-Z (SEQ ID No. 28), U-KIGD-Z (SEQ ID No. 29), U-KRGD-Z (SEQ ID No. 30), U-LEGD-Z (SEQ ID No. 31), U-LKGD-Z (SEQ ID No. 32), U-LAGD-Z (SEQ ID No. 33), U-LIGD-Z (SEQ ID No. 34), U-LRGD-Z (SEQ ID No. 35), U-IEGD-Z (SEQ ID No. 36), U-IKGD-Z (SEQ ID No. 37), U-ILGD-Z (SEQ ID No. 38), U-IAGD-Z (SEQ ID No. 39), U-IRGD-Z (SEQ ID No. 40), U-REGD-Z (SEQ ID No. 41), U-RKGD-Z (SEQ ID No. 42), U-RLGD-Z (SEQ ID No. 43), U-RAGD-Z (SEQ ID No. 44), U-RIGD-Z (SEQ ID No. 45), U-AEGD-Z (SEQ ID No. 46), U-AKGD-Z (SEQ ID No. 47), U-ALGD-Z (SEQ ID No. 48), U-AIGD-Z (SEQ ID No. 49), U-ARGD-Z (SEQ ID No. 50).

In another embodiment, the tetrapeptide is selected from the group consisting of U-LSVD-Z (SEQ ID No. 5), U-LSPD-Z (SEQ ID No. 12) and U-LSPG-Z (SEQ ID No. 13). In another embodiment the tetrapeptide is Pal-LSVD-OH (SEQ ID No. 5). In another embodiment the tetrapeptide is Pal-LSPD-OH (SEQ ID No. 12). In another embodiment the tetrapeptide is Pal-LSPG-OH (SEQ ID No. 13).

In another embodiment, the tetrapeptide is selected from the group consisting of U-EKGD-Z (SEQ ID No. 21), U-LKGD-Z (SEQ ID No. 32), U-IRGD-Z (SEQ ID No. 40) and U-AKGD-Z (SEQ ID No. 47). In another embodiment the tetrapeptide is U-EKGD-Z (SEQ ID No. 21). In another embodiment the tetrapeptide is U-LKGD-Z (SEQ ID No. 32). In another embodiment the tetrapeptide is U-IRGD-Z (SEQ ID No. 40). In another embodiment the tetrapeptide is U-AKGD-Z (SEQ ID No. 47).

In another embodiment, the tetrapeptide is selected from the group consisting of U-GPKG-Z (SEQ ID No. 17), U-GPEG-Z (SEQ ID No. 18) and U-GPSG-Z (SEQ ID No. 19).

Where, at the N-terminal, U is H then the amino acid is not modified. When, at the C-terminal, Z is OH then the amino acid is not modified. The tetrapeptide is thus not in derivatised form. When other than U is H and Z is OH, then the tetrapeptide is derivatised. Derivation of the tetrapeptide is intended to increase the bioavailability of the peptide by improving the ability of the tetrapeptide to pass through the skin. An increase in bioavailability can also be achieved through vectoring, for example by encapsulation of the peptide.

In a preferred embodiment, the tetrapeptide is modified at the N-terminal and/or the C-terminal end.

In a preferred embodiment, R¹ and/or R² is an alkyl chain of from 1 to 24 carbon atoms, preferably a lipophilic alkyl chain of 3 to 24 carbon atoms.

In a further preferred embodiment, U is an acyl group -CO-R¹ and Z is selected from the group consisting of OH, methoxy, ethoxy and NH₂, preferably OH. In a further embodiment, U is preferably independently selected from the group consisting of octanoyl (C8), decanoyl (C10), lauroyl (C12), myristoyl (C14), palmitoyl (C16), stearoyl (C18), biotinoyl, elaidoyl, oleoyle and lipoyle. In a preferred embodiment U is independently selected from lauroyl (C12), myristoyl (C14) and palmitoyl (C16).

In a further preferred embodiment, Z is OH and U is independently selected from the group consisting of palmitoyl (C16), myristoyl (C14) and lauroyl (C12). Most preferably U is palmitoyl (C16) and Z is OH.

The tetrapeptides may comprise amino acids in the D- or L- configuration. The tetrapeptides may comprise an acid C-terminus such as -CO₂H.

The amino acids making up the tetrapeptides according to the invention may be optically pure, be made up of L or D isomers or a mixture thereof. L isomers are those present in the natural state and may be preferred.

Further embodiments also envisage and include further derivatives of the tetrapeptide, including for example modification and/or addition of a chemically functional group to one or more of the amino acids but without a change in the carbon skeletal. The invention also envisages and includes further analogues of the tetrapeptide, including modification and/or addition of a chemically functional group to one or more of the amino acids with a change in the carbon skeletal and complexes of the tetrapeptide with other species such as a metal ion (e.g. copper, zinc, manganese, magnesium, and others).

Tetrapeptides of the invention may be present in the form of salts, including hydrochloric salt, or acetate.

In some embodiments, the tetrapeptide is a combination of two or more tetrapeptides, for example, three or more, four or more, or five or more selected from the list consisting of:
a) tetrapeptides having the amino acid sequence U-LSXX-Z (SEQ ID No. 1) wherein L is used to denote amino acid Leucine and S is used to denote Serine, as per the internationally recognised single letter code for amino acids and X denotes an amino acid selected from the group consisting of Valine (V), Aspartic acid (D), Proline (P), Glycine (G) and mixtures thereof;
b) a tetrapeptide having the amino acid sequence U-GPXG-Z (SEQ ID No. 2) wherein G is used to denote amino acid glycine and P denotes the amino acid proline, as per the internationally recognised single letter code for amino acids, X denotes an amino acid independently selected from the group consisting of Lysine (K), Glutamic acid (E), Serine (S) and mixtures thereof;
c) tetrapeptides having the amino acid sequence U-XXGD-Z (SEQ ID No. 3) wherein G is used to denote amino acid Glycine and D is used to denote amino acid Aspartic acid, as per the internationally recognised single letter code for amino acids and X denotes an amino acid selected from the group consisting of Glutamic acid (E), Lysine (K), Leucine (L), Alanine (A), Isoleucine (I), Arginine (R) and mixtures thereof; and
d) tetrapeptides having the amino acid sequence U-QTAV-Z (SEQ ID No. 4) wherein Q is used to denote amino acid Glutamine, T is used to denote amino acid Threonine, A is used to denote amino acid Alanine and V is used to denote amino acid Valine, as per the internationally recognised single letter code for amino acids.

In some preferred embodiments the tetrapeptide is a combination of two or more tetrapeptides, wherein a first peptide is selected from group a) and a second peptide is selected from group b) as outlined above. For example, the tetrapeptide is a combination of a first tetrapeptide selected from the group consisting of U-LSVD-Z (SEQ ID No. 5), U-LSPD-Z (SEQ ID No. 12) and U-LSPG-Z (SEQ ID No. 13), and the second tetrapeptide is selected from the group consisting of U-GPKG-Z (SEQ ID No. 17), U-GPEG-Z (SEQ ID No. 18) and U-GPSG-Z (SEQ ID No. 19).

In other preferred embodiments the tetrapeptide is a combination of two or more tetrapeptides, wherein a first peptide is selected from group c) and a second peptide is selected from group b) as outlined above. For example, the tetrapeptide is a combination of a first tetrapeptide selected from the group consisting of U-EKGD-Z (SEQ ID No. 21), U-LKGD-Z (SEQ ID No. 32), U-IRGD-Z (SEQ ID No. 40) and U-AKGD-Z (SEQ ID No. 47), and the second tetrapeptide is selected from the group consisting of U-QTAV-Z (SEQ ID No. 4), U-GPKG-Z (SEQ ID No. 17), U-GPEG-Z (SEQ ID No. 18) and U-GPSG-Z (SEQ ID No. 19).

In other preferred embodiments the tetrapeptide is a combination of two or more tetrapeptides, wherein a first peptide is selected from group d) and a second peptide is selected from group b) as outlined above. For example, the first tetrapeptide is U-QTAV-Z (SEQ ID No. 4) and the second tetrapeptide is selected from the group consisting of U-GPKG-Z (SEQ ID No. 17), U-GPEG-Z (SEQ ID No. 18) and U-GPSG-Z (SEQ ID No. 19).

In a most preferred embodiment, the tetrapeptide is a combination selected from the group consisting of combinations of U-LSVD-Z (SEQ ID No. 5) and U-GPKG-Z (SEQ ID No. 17), U-QTAV-Z (SEQ ID No. 4) and U-GPKG-Z (SEQ ID No. 17), U-QTAV-Z (SEQ ID No. 4) and U-EKGD-Z (SEQ ID No. 21), and U-LSVD-Z (SEQ ID No. 5) and U-GPKG-Z (SEQ ID No. 17) and U-QTAV-Z (SEQ ID No. 4) and U-EKGD-Z (SEQ ID No. 21).

The combination of tetrapeptides in one embodiment can show synergistic benefit in that they offer markedly improved levels of fibrillin-1 production in human dermal fibroblast cells in comparison the peptides singularly.

### Tetrapeptide of the Second, Third, Fourth, Fifth and Sixth Aspects

The invention of the second, third, fourth, fifth and sixth aspects comprises a tetrapeptide, specific embodiments of which are now described.

With respect to the the invention of the third, fourth, fifth and sixth aspects of the invention, the tetrapeptide may be selected from the group comprising RSRK (SEQ ID No. 51) and U-GQPR (SEQ ID No. 52) peptides, wherein R is used to denote amino acid Arginine, S is used to denote amino acid Serine, K is used to denote amino acid Lysine, G is used to denote amino acid Glycine, Q is used to denote amino acid Glutamine, and P is used to denote the amino acid proline as per the internationally recognised single letter code for amino acid. Wherein at the N-terminal end of the one or more tetrapeptide, U is selected from the group consisting of H, -CO-R¹, -SO₂-R¹ or a biotinyl group, and wherein R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N. The tetrapeptide of the second aspect is selected from this group.

Where, at the N-terminal, U is H then the amino acid is not modified. The tetrapeptide is thus not in derivatised form. When other than U is H, then the tetrapeptide is derivatised. Derivation of the tetrapeptide is intended to increase the bioavailability of the peptide by improving the ability of the tetrapeptide to pass through the skin. An increase in bioavailability can also be achieved through vectoring, for example by encapsulation of the peptide.

In a preferred embodiment, the tetrapeptide is modified at the N-terminal.

In a preferred embodiment, R¹ and/or R² is an alkyl chain of from 1 to 24 carbon atoms, preferably a lipophilic alkyl chain of 3 to 24 carbon atoms.

In a further preferred embodiment, U is an acyl group -CO-R¹. In a further embodiment, U is preferably independently selected from the group consisting of octanoyl (C8), decanoyl (C10), lauroyl (C12), myristoyl (C14), palmitoyl (C16), stearoyl (C18), biotinoyl, elaidoyl, oleoyle and lipoyle. In a preferred embodiment, U is independently selected from lauroyl (C12), myristoyl (C14) and palmitoyl (C16).

Further embodiments also envisage and include further derivatives of the tetrapeptide, including for example modification and/or addition of a chemically functional group to one or more of the amino acids but without a change in the carbon skeletal. The invention also envisages and includes further analogues of the tetrapeptide, including modification and/or addition of a chemically functional group to one or more of the amino acids with a change in the carbon skeletal and complexes of the tetrapeptide with other species such as a metal ion (e.g. copper, zinc, manganese, magnesium, and others).

Tetrapeptides, may be present in the form of salts, including hydrochloric salt, or acetate.

In one embodiment of the second, third, fourth, fifth and sixth aspects, the tetrapeptide is N-palmitoyl-Gly-Gln-Pro-Arg.

### Matrix Metalloproteinase Inhibitors (MMPi)

In some embodiments of the invention, the cosmetic composition or kit comprises matrix metalloproteinase inhibitors (MMPi). In one embodiment compositions of the invention comprising tetrapeptide further comprise one or more matrix metalloproteinase inhibitor. The presence of one or more matrix metalloproteinase inhibitor in the cosmetic compositions of the invention comprising tetrapeptide enhances protein synthesis, skin repair and regeneration.

The term "matrix metalloproteinase inhibitor" relates to all molecule and/or plant or bacterial extracts having an inhibitory activity on at least one of the matrix metalloproteinases expressed or synthetized by or in the skin. The family of the matrix metalloproteinases is formed of several well-defined groups on the basis of their resemblance regarding structure and substrate specificity (Woessner J. F. 1991, Faseb Journal, vol. 5,, 2145). Among these groups, there are collagenases able to degrade fibrillar collagens (MMP-1 or interstitial collagenase, MMP-8 or neutrophil collagenase, MMP- 13 or collagenase 3, MMP-18 or collagenase 4), gelatinases degrading type IV collagen or other denatured collagen form (MMP-2 or A gelatinase (72 kDa), MMP-9 or B gelatinase (92 kDa)), stromelysins (MMP-3 or stromelysin 1, MMP- 10 or stromelysin 2, MMP-11 or stromelysin 3) whose broad spectrum of activity targets proteins of the dermal extracellular matrix such as glycoproteins (fibronectin, laminin), proteoglycanes etc., matrilysin (MMP-7), metalloelastase (MMP- 12) or metalloproteinases (MMP- 14, MMP- 15, MMP- 16 and MMP- 17). Metalloproteinases (MMPs) are proteases that use a metal, (mostly zinc) coordinated to 3 cysteine residues and to a methionine in their active site, that degrade macromolecular components of the dermal extracellular matrix and of basal layers at neutral pH (collagen, elastin, etc.). This group of enzymes is inactivated by metal chelators. The principal activity regulators of MMPs are the tissue inhibitors of metalloproteinases or TIMPs such TIMP-I, TIMP-2, TIMP-3 and TIMP-4 (Woessner J. F., Faseb Journal, 1991). Furthermore, MMP expression is also regulated by growth factors, cytokines, oncogene products (ras, jun), or also matrix constituents.

The term "matrix metalloproteinase inhibitors" according to the invention means all molecules able to reduce the MMP's activity regarding the gene expression (transcription and translation) or regarding the activation of the zymogen form of the MMP, or else regarding the local control of active forms. Furthermore, the metalloproteinase inhibitors according to the invention can also be MMP-1 inhibitors of natural or synthetic origin. The terms "natural origin" or "synthetic origin" mean both a metalloproteinase inhibitor at a pure state or in solution at different concentrations, but natural origin termed inhibitors are obtained by different extraction methods from a natural element (for example lycopene from a tomato) whereas the inhibitors of synthetic origin are all obtained via chemical synthesis

In one embodiment, MMPi are selected from the group consisting of retinoid, N-acetyl cysteine, glutathione, 2-furildioxime, vitamin C, flavones, isoflavones, hydrolysed rice protein, alfalfa extract, white lupin, zizyphus jujube extract, dihydroxy methyl chromone, kudzu extract, vitis vinifera extract, oenothera biennis extract, anogeissus leiocarpus extract, soyabean extract, phyllanthus emblica extract, lentinus edodes extract, argania spinosa extract, paeonia lactiflora extract, pisum sativum extract, cistus monspellensis extract, litchi chinensis pericarp extract, plantago lanceolata extract, salicyloyl phytosphingosine, cyanidium caldarium extract, lactobionic acid, stevia rebaudiana extract, acetyl tripeptide-30 citrulline, morus alba extract, and mixtures thereof.

Where present, MMPi are present at a level of from 0.001% to 10%, more preferably 0.01% to 5% and most preferably from 0.05% to 2.5% by weight of the cosmetic composition.

### Additional Peptides

The cosmetic compositions of the invention may comprise further peptides. Preferably said additional peptides are selected from the group consisting of dipeptides, tripeptides, additional tetrapeptides, pentapeptides and mixtures thereof. By tripeptides, it is meant compound comprising an uninterrupted sequence of three amino acids. By tetrapeptides, it is meant a compound comprising an uninterrupted sequence of four amino acids and when using further tetrapeptides, the tetrapeptides are referred to as 'additional tetrapeptides'. By pentapeptide it is meant a compound comprising an uninterrupted sequence of five amino acids.

### Dipeptides

The cosmetic compositions of the invention may comprise a dipeptide selected from the group consisting of acetyl dipeptide 1 cetyl ester, acetyl dipeptide 3 aminohexanoate, azelaoyl bisdipeptide 10, coumaroyl dipeptide 3, dicetyl dipeptide 9, dipeptide diamino butyroyl benzylamide diacetate, dipeptide 1, dipeptide 10, dipeptide 11, dipeptide 12, dipeptide 15, dipeptide 16, dipeptide 17, dipeptide 18, dipeptide 19, dipeptide 2, dipeptide 20, dipeptide 3, dipeptide 4, dipeptide 5, dipeptide 6, dipeptide 7, dipeptide 8, dipeptide 8 HCL, dipeptide 9, hexanoyl dipeptide 3 norleucine acetate, methyl undecylenoyl dipeptide 16, nicotinoyl dipeptide 22, nicotinoyl dipeptide 23, nicotinoyl dipeptide 24, nicotinoyl dipeptide 26, oleoyl dipeptide 15, palmitoyl dipeptide 10, palmitoyl dipeptide 13, palmitoyl dipeptide17, palmitoyl dipeptide 5 diaminobutyroyl hydroxythreonine, palmitoyl dipeptide 5 diaminohydroxybutyrate, palmitoyl dipeptide 7 and mixtures thereof.

Dipeptides are preferably incorporated into the cosmetic compositions of the invention at a level of from 0.1 to 50000ppm, more preferably from 1 to 5000 ppm, most preferably from 10 to 500ppm.

### Tripeptides

The cosmetic compositions of the invention preferably comprise a tripeptide. Said tripeptide may be naturally occurring or of synthetic origin. Suitable tripeptides include tripeptide 1, 2, 3, 4, 5, 6, 7, 8, 9, 10,11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, derivatives thereof and mixtures thereof.

Particularly preferred tripeptides comprise one or more His-based tripeptides. However, another suitable tripeptide may be Arg-Lys-Arg. Particularly preferred tripeptides are based on the structure Gly-His-Lys and its analogs and derivatives thereof. These are collectively known herein as GHK-tripeptides. Indeed, the preferred tripeptide in accordance with this aspect of the invention has this exact sequence of amino acids. Analogs of the preferred tripeptide useful herein include those in which one or more of the three amino acids are reorganized or rearranged within the sequence (e.g., Gly-Lys-His) and/or where no more than two amino acids are substituted (e.g., His-Ala-Orn). However, most preferably, amino acids substituted for Gly include an aliphatic side chain such as, without limitation, beta-Ala, Ala, Val, Leu, Pro, Sarcosine (Sar) and Ile. Most preferred are Ala, Leu and Ile. The most preferable amino acid substituted for Lys or His include those having a side chain that includes, predominantly, a charged nitrogen at a pH of 6, such as, without limitation, Pro, Lys, Arg, His, Desmosine and Isodesmosine. Most preferably, Lys is replaced with Orn, Arg, or Citrulline.

Derivatives are also considered to be encompassed by the term GHK-tripeptides in accordance with the invention, (and therefore also the more generic term tripeptides). Derivatives of GHK-tripeptides in accordance with the invention include derivatives of the substituted and rearranged tripeptides described herein. These derivatives include, inter alia, acyl-derivatives, which are tripeptides substituted with one or more straight-chain or branched-chain, long or short chain, saturated or unsaturated, substituted with a hydroxy, amino, acyl amino, sulfate or sulfide group, or unsubstituted, which can be derived from acetic acid, capric acid, lauric acid, myristic acid, octanoic acid, palmitic acid, stearic acid, behenic acid, linoleic acid, linolenic acid, lipoic acid, oleic acid, isostearic acid, elaidoic acid, 2-ethylhexaneic acid, coconut oil fatty acid, tallow fatty acid, hardened tallow fatty acid, palm kernel oil fatty acid, lanolin fatty acid and the like. Preferable examples of the acyl group include an acetyl group, a palmitoyl group, an elaidoyl group, a myristyl group, a biotinyl group and an octanoyl group. These may be substituted or unsubstituted. When substituted, they are preferably substituted with hydroxyl or sulphur comprising groups such as, without limitation SO₃H, SH or S-S.

His-based tripeptides include at least one histidine amino acid. The other two amino acids in the sequence may be the same or different. Thus, contemplated are, without limitation, His-Xaa-Xaa, His-Xaa-Xbb, His-Xbb-Xaa, Xbb-His-Xbb, Xbb-His-Xaa, Xaa-His-Xbb, Xaa-Xaa-His, Xaa-Xbb-His, Xbb-Xaa-His and Xbb-Xbb-His, where Xaa and Xbb are two different amino acids, although either can be His. Preferably, at least one of the other amino acids is Gly, beta-Ala, Ala, Val, Leu, Pro, Sarcosine (Sar) or Ile. Preferably, at least one of the other amino acids is Pro, Lys, Arg, His, Desmosine and Isodesmosine. Most preferably, Lys is replaced with Orn, Arg, or Citrulline.

Derivatives are also considered to be encompassed by the term His-based tripeptides in accordance with the invention, (and therefore also the more generic term tripeptides). These derivatives include, inter alia, acyl-derivatives, which are tripeptides substituted with one or more straight-chain or branched-chain, long or short chain, saturated or unsaturated substituted or unsubstituted acyl group(s) having from 1 to 29 carbon atoms. The acyl groups which can be used are the same as those described for the GHK-tripeptides.

Particularly preferred embodiments of tripeptides in accordance with the invention include N-Acyl-Gly-His-Lys and most preferably, N-Palmitoyl-Gly-His-Lys. Preferred commercially available tripeptide and tripeptide derivative comprising compositions include Biopeptide-CL from SEDERMA, Maxilip(R) from SEDERMA, Biobustyl(R) from SEDERMA.

The tripeptides where included are preferably incorporated into the cosmetic composition in amounts of from 0.10ppm to 10,000ppm, preferably from 0.50ppm to 5,000ppm, more preferably from 1ppm to 1000ppm, and most preferably from 1ppm to 500ppm. These are again based on a % w/w basis. Thus 100,000ppm is 10% by weight of the cosmetic composition.

### Additional Tetrapeptides

The cosmetic composition may comprise an additional tetrapeptide. These may be one or more rigin-based tetrapeptides, one or more ALAMCAT-tetrapeptides or mixtures thereof. These tetrapeptides may be naturally occurring or of synthetic origin. Suitable tetrapeptides for use in the cosmetic compositions of the invention include those selected from the group consisting of well-known tetrapeptide 1, 2, 3, 4, 5, 6, 7, 8, 9, 10, 11, 12,13, 14, 15, 16, 17, 18, 19 ,20, 21, 22, 23, 24, 25, 26, 27, 28, 29, 30, 34, 35, derivatives thereof and mixtures thereof.

Rigin-based tetrapeptides in accordance with the invention are based on the structure Gly-Gln-Pro-Arg (Rigin) and include its analogs and derivatives thereof. Rigin is an additional tetrapeptide. Analogs of the tetrapeptide rigin useful in accordance with the invention include those in which one or more of the four amino acids are reorganized or rearranged within the sequence and/or where no more than two of the amino acids are substituted (e.g., Ala-Gln-Thr-Arg. More preferably, at least one of the amino acids within the sequence is Pro or Arg and most preferably the tetrapeptide includes both Pro and Arg although their order and position may vary. The amino acid substitutions can be from amongst any amino acid as defined herein. Particularly preferred rigin-based tetrapeptides include Xaa-Xbb-Arg-Xcc, Xaa-Xbb-Xcc-Pro, Xaa-Xbb-Pro-Arg, wherein Xaa-Xbb-Pro-Xcc, Xaa-Xbb-Xcc-Arg, Xaa, Xbb and Xcc may be the same or different and selected from the following Xaa is Gly or the amino acids that may be substituted therefore, Xbb is Gln or the amino acids that may be substituted therefore and Xcc may be Pro or Arg or the amino acids substituted therefore. The most preferable amino acids substituted for Gly include an aliphatic side chain such as, without limitation, beta-Ala, Ala, Val, Leu, Pro, Sarcosine (Sar) and Ile. The most preferable amino acids substituted for Gln include a side chain that includes an amine group that is predominantly uncharged at neutral pH (pH 6-7) such as, without limitation, Asn, Lys, Orn, 5-hydroxyproline, Citrulline and Canavanine. When Arg is substituted, it is preferably replaced with an amino acid having a side chain that includes, predominantly, a charged nitrogen at a pH of 6, such as, without limitation, Pro, Lys, His, Desmosine and Isodesmosine.

Derivatives are also considered to be encompassed by the term rigin-base tetrapeptides, (and therefore also the more generic term tetrapeptides). Derivatives include derivatives of the substituted and rearranged rigin-based tetrapeptides described herein. These derivatives include, inter alia, acyl-derivatives, which are tetrapeptides substituted with one or more straight-chain or branched-chain, long or short chain, saturated or unsaturated, substituted with a hydroxy, amino, amino acyl, sulfate or sulfide group or unsubstituted having from 1 to 29 carbon atoms. N-acyl-derivatives include those acyl groups which can be derived from acetic acid, capric acid, lauric acid, myristic acid, octanoic acid, palmitic acid, stearic acid, behenic acid, linoleic acid, linolenic acid, lipoic acid, oleic acid, isostearic acid, elaidoic acid, 2-ethylhexaneic acid, coconut oil fatty acid, tallow fatty acid, hardened tallow fatty acid, palm kernel oil fatty acid, lanolin fatty acid and the like. Preferable examples of the acyl group include an acetyl group, a palmitoyl group, an elaidoyl group, a myristyl group, a biotinyl group and an octanoyl group. These may be substituted or unsubstituted. When substituted, they are preferably substituted with hydroxyl or sulphur comprising groups such as, without limitation SO₃H, SH or S-S.

Derivatives are also considered to include peptide-divalent ion complexes. Cu²⁺-peptide derivatives are preferred as this may provide increased biological effect compared to the peptide alone.

ALAMCAT tetrapeptides are tetrapeptides which include at least one amino acid including an aliphatic group comprising side chain. These amino acids include, without limitation, Gly, beta-Ala, Ala, Val, Leu, Sarcosine (Sar) and Ile. These tetrapeptides also include at least one amino acid including at least one NH₂ comprising side chain. These amino acids include a side chain that has an amine group that is predominantly uncharged at neutral pH (pH 6-7) such as, without limitation, Gin, Asn, Lys, Orn, 5-hydroxyproline, Citrulline and Canavanine. The ALAMCAT-tetrapeptides also include at least one amino acid having at least one side chain including at least one cationic amine (predominant species is charged such as NH₃⁺, NH₂⁺, etc.-basic amino acids which are positively charged at pH 6.0). These amino acids include, without limitation, Pro, Arg, Lys, His, Desmosine and Isodesmosine. The remaining amino acid can be any amino acid, but is preferably one comprising an alphatic group, pendant amino group or pendant cationic group. Derivatives are also considered to be encompassed by the term ALAMCAT-tetrapeptides in accordance with the invention, (and therefore also the more generic term tetrapeptides). These derivatives include, inter alia, acyl-derivatives, which are tetrapeptides substituted with one or more straight-chain or branched-chain, substituted or unsubstituted long or short chain, saturated or unsaturated acyl group(s) having from 1 to 29 carbon atoms. The acyl groups which can be used are the same as those described for the rigin-based tetrapeptides.

Preferred embodiments include Peptide E, arg-ser-arg-lys, N-acyl-Gly-Gln-Pro-Arg peptides, most preferably N-palmitoyl-Gly-Gln-Pro-Arg.

Preferred commercially available sources of tetrapeptides include RIGIN, EYELISS, Haloxyl, and MATRIXYL 3000, which comprise between 50 to 500 ppm of palmitoyl-Gly-Gln-Pro-Arg, and other ingredients, such as peptides, chalcones and an excipient, commercially available from SEDERMA, France. Tego Pep 417 available from Evonik. These may be used to produce compositions of the invention by adding thereto at least one tripeptide as described herein.

The additional tetrapeptides when used are preferably incorporated into the cosmetic composition in amounts from 0.1 ppm (0.00001% w/w also referred to herein as "weight percent", "weight %" or simply by weight) to 10,000 ppm (1.0% w/w), preferably from 0.5 ppm to 1000 ppm (0.1% w/w), and most preferably from 1 ppm to 500ppm (0.05% w/w) by weight of the cosmetic composition.

The combination of tripeptides and additional tetrapeptides, can be particularly preferred. When present, the preferred ratio of additional tetrapeptide to tripeptide, or indeed the ratio of molecules having four amino acids to those having three amino acids can range from 100:1 to 1:100; more preferably from 50:1 to 1:50, even more preferably from 30:1 to 1:30 and even more preferably between 10:1 to 1:10. Most preferably, the ratio of additional tetrapeptide to tripeptide ranges from between 3:1 to 1:3. These ratios are on a weight basis (% w/w-e.g. mg of pure peptide per Kilogram in the final formulation). In a particularly preferred embodiment, the amount of tripeptide used is greater than the amount of additional tetrapeptide used when considered in terms of their amounts in parts per million, again based on overall weight of the cosmetic composition. In a particularly preferred embodiment, the cosmetic composition of the invention comprises an additional tetrapeptide of the sequence Gly-Gln-Pro-Arg, its analogs and derivatives in combination with one or more tripeptide of the sequences Gly-His-Lys, its analogs and derivatives.

### Pentapeptides

The cosmetic compositions of the invention may optionally comprise a pentapeptide, derivatives of pentapeptides, and mixtures thereof. As used herein, "pentapeptides" refers to both the naturally occurring pentapeptides and synthesized pentapeptides. Also, useful herein are naturally occurring and commercially available compositions that comprise pentapeptides. Suitable pentapeptides are those selected from the group consisting of pentapeptide1, 4, 5, 6, 7, 8, 9, 10, 11, 12, 13, 14, 15, 16, 17, 18, 19, 21, 22, 23, 24, 25, 26, 28, 29, 30, 31, 33, 34, 35, 36, 38, 39, derivatives thereof and mixtures thereof.

Suitable pentapeptides for use herein are the pentapeptide, lys-thr-thr-lys-ser, Arg-asp-lys-tyr-val (pentapeptide -1) and derivatives thereof. A preferred commercially available pentapeptide derivative-comprising composition is Matrixyl which comprises 100 ppm of palmitoyl-lys-thr-thr-lys-ser and is commercially available from Sederma, France.

The pentapeptides when used are preferably incorporated into the cosmetic compositions in amounts from 0.1 ppm (0.00001% w/w also referred to herein as "weight percent", "weight %" or simply by weight) to 10,000 ppm (1.0% w/w), preferably from 0.5 ppm to 1000 ppm (0.1% w/w), and most preferably from 1 ppm to 500ppm (0.05% w/w) by weight of the cosmetic composition.

### Other Components

The compositions of the invention may be aqueous or non-aqueous and comprise of a single-phase system or multiple phase system. The cosmetic compositions may include but is not limited to liquids, gels, balms, oils or solids. Single or multiple phase compositions are envisaged. Multiple phase systems include but are not limited to microemulsions, emulsions, and products with discrete separate phases. Emulsions include water-in-oil, oil-in-water emulsions and multiple emulsions (water in oil in water or oil in water in oil for example). Products with discrete separate phases include bi or triphasic systems where the individual water or oil phases can be visibly seen.

Where the cosmetic composition is aqueous, it preferably comprises from 10% to 99.9% by weight water. In a preferred embodiment, aqueous compositions comprise from 20% to 80 % by weight water. In a preferred embodiment, aqueous compositions comprise from 40% to 70% by weight water. Where the composition is non-aqueous it preferably comprises 0% to up to 10% water, more particularly from 0.1 to 8%, most preferably from 0.5 to 5% water.

Where the cosmetic composition is an emulsion it comprises an oil and a water phase. The oil phase of an emulsion can be provided by any suitable oily component. Suitable oils for the oil phase may comprise for example: a) hydrocarbon oils, such as paraffin or mineral oils; b) waxes, such as beeswax or paraffin wax; c) natural oils, such as sunflower oil, apricot kernel oil, shea butter or jojoba oil; d) silicone oils, such as dimethicone, silicone elastomer, cyclomethicone or cetylidimethicone; e) fatty acid esters and ethers, such as isopropyl palmitate or isopropyl myristate and polypropylene glycol-15 stearyl ether; f) fatty alcohols, such as cetyl alcohol or stearyl alcohol; or g) mixtures thereof, for example, the blend of waxes available commercially under the trade name Cutina (BASF).

The emulsion may comprise 0.1% to 55% by weight of the emulsion of oil phase. In one embodiment, the emulsion may comprise 3% to 25% by weight of the emulsion of oil phase, more preferably from 5% to 20% by weight of the emulsion of oil phase. In an alternative embodiment, the emulsion may comprise 10% to 50% by weight of the emulsion of oil phase, more preferably from 25-50% by weight of the emulsion of oil phase.

The cosmetic compositions of the invention may comprise an emulsifier. Suitable emulsifiers include all those suitable for the purpose and known by those skilled in the art for use in skin care products. Preferably these emulsifiers have an HLB value of or less than 14, more preferably from 2 to 14, and still more preferably from 4 to 14.

In some embodiments, the cosmetic compositions further comprise one or more antioxidant agents.

Suitable antioxidant agents may include: a) ascorbic acid its salts, esters, glucosides and glucosamines, particularly sodium ascorbyl phosphate, magnesium ascorbyl phosphate, ascorbyl palmitate and ethyl ascorbic acid b) vitamin E (tocopherol) and its esters, particularly tocopheryl acetate, as well as Dimethyl methoxy chromanol which is a synthetic analogue of gamma tocopherol, available from Lipotec S.A. polygon Industrial Camri Ral, under the tradename Lipochroman-6 c) herbal extracts, particularly gingko biloba, such as that available under the trade name "Gingko Biloba Leaf Powder" from Univar PLC, morus alba, such as that available under the trade name "Mulberry Concentrate" from Solabia, origanum vulgare, such as that available under the trade name "Pronalen Origanum HSC" from S Black Ltd, panax ginseng, such as that available under the trade name "Panax ginseng 1.1 extract 4294"from S Black Ltd or "Phytexcell Panax ginseng" available from Croda Chemicals Ltd, birch extract such as those available from Cosmetochem (U. K.) Ltd under the trade names "Super Herbasol Extract Birch" and "HP Herbasol Betula" and those available from Blagden Chemicals under the tradenames "Phytelene of Birch" and "Aqueous Spray Dried Birch", camellia sinensis, such as that available under the trade name "Herbal Extract Green Tea 75% Solids" from Nichimen Europe, rosmarinus officinalis, such as that available under the trade name "Pronalen Rosemary" from S. Black, Acerola cherry powder, such as that available as Acerola PE from Gee Lawson, Emblica extract sold under the tradename Emblica^{™} by Merck Speciality chemicals, and Grape Seed oil, such as that available from Chesham Chemicals Limited.

The amounts of antioxidant agents used in the cosmetic compositions are expressed as dry weights, as understood by a man skilled in the art. The total amount of antioxidant agents optionally present in the cosmetic compositions may range from 0.005 % to 10 % by weight, preferably 0.01 % to 5 %, most preferably 0.05 % to 1.0 % by weight of the total weight of the cosmetic composition.

The cosmetic compositions of the invention may optionally comprise a skin conditioning agent. Said skin conditioning agents may preferably be selected from the group consisting of humectants, emollients, moisturisers, or mixtures thereof. Where present, they are preferably present at a level of from 0.01% to 20%, more preferably from 0.1% to 10%, most preferably from 0.5% to 7% by weight of the cosmetic composition.

Preferred skin conditioning agents are selected from the group consisting of guanidine, urea, glycolic acid and glycolate salts, salicylic acid, lactic acid and lactate salts, aloe vera, shea butter, polyhydroxy alcohols, such as sorbitol, mannitol, xylitol, erythritol, glycerol, hexanetriol, butanitriol, (di) propylene glycol, butylene glycol, hexylene glycol, polyethylene glycol, sugars (e.g. fructose, glucose, xylose, honey, mannose, xylose), gluconodeltalactone, and starches and their derivatives, pyrrolidone, carboxylic acid, hyaluronic acid and salts thereof, lactamide monoethanolamine, acetamide monoethanolamine, panthenol, allantoin and mixtures thereof.

The cosmetic compositions of the invention may comprise one or more vitamins. The cosmetic compositions may comprise ascorbates, for example vitamin C, vitamin C derivatives, ascorbic acid, ascorbyl glucoside, ascorbyl palmitate, magnesium ascorbyl phosphate, sodium ascorbyl phosphate and ethyl ascorbic acid. The cosmetic compositions may comprise vitamin B, vitamin B derivatives, vitamin B1 to vitamin B12 and their derivatives. In a further embodiment the cosmetic compositions comprising the Vitamin B3 derivative niacinamide.

In some embodiments the cosmetic compositions may comprise vitamin K, vitamin K derivatives, vitamin H, vitamin D, vitamin D derivatives and mixtures thereof. In an alternative embodiment of the present the cosmetic compositions comprise vitamin E, vitamin E derivatives such as tocopherol and tocopheryl acetate, and provitamins thereof, such as panthenol and mixtures thereof.

In a further embodiment, the cosmetic compositions of the invention comprise retinoid compounds, including retinoic acid, retinaldehyde, retinol and derivatives thereof. In one embodiment the cosmetic compositions comprise retinyl palmitate, retinyl acetate, retinyl retinoate, retinyl proprionate, retinyl ascorbate, retinyl linoleate, retinyl retinoate, retinyl sunflowerseedate and mixtures thereof.

The vitamin compounds may be included as the substantially pure material, or as an extract obtained by suitable physical and/or chemical isolation from natural (e.g. plant) sources. In one embodiment, when vitamin compounds are present in the cosmetic compositions of the instant invention, the cosmetic compositions comprise from about 0.0001% to 50%, more preferably from 0.001% to 10%, still more preferably from 0.01% to 8%, and still more preferably from 0.1% to 5%, by weight of the cosmetic composition, of the vitamin compound.

The cosmetic compositions of the invention may optionally comprise a sunscreen component. The sunscreen may comprise organic or inorganic sun filters or a combination of the two. Suitable inorganic sun filters include those selected from the group consisting of microfine titanium dioxide, microfine zinc oxide, boron nitride and mixtures thereof. Suitable organic sunscreens include those selected from the group consisting of: a) p-aminobenzoic acids, their esters and derivatives (for example, 2ethylhexyl p-dimethylaminobenzoate), b) methoxycinnamate esters (for example, 2-ethylhexyl p-methoxycinnamate, 2-ethoxyethyl p-methoxycinnamate or a, p-di- (p-methoxycinnamoyl)-a'- (2ethylhexanoyl)-glycerin, c) benzophenones (for example oxybenzone), d) dibenzoylmethanes such as 4-(tert-butyl)-4'-methoxydibenzoylmethane, e) 2-phenylbenzimidazole-5 sulfonic acid and its salts, f) alkyl-ss, ss-diphenylacrylates for example alkyl a-cyano-ss, ss-diphenylacrylates such as octocrylene, g) triazines such as 2,4,6-trianilino-(p-carbo-2-ethyl-hexyl-1-oxi)-1, 3,5-triazine, h) camphor derivatives such as methylbenzylidene camphor and i) mixtures thereof. Other preferred sunscreen ingredients include those selected from the group consisting of homosalate, Ethylhexyl salicylate, Diethylhexylbutamido triazone, Bis-ethylhexyloxyphenol methoxyphenyl triazine, Diethylamino hydroxybenzoyl hexyl benzoate, Butyl methoxydibenzoylmethane, Methylene bis - benzotriazoyl tetramethylbutylphenol, Polysilicone-15 and mixtures thereof. A sunscreen agent is optionally present in an amount from 0.1 to 10% by weight of the cosmetic composition.

The cosmetic compositions of the invention may also optionally comprise one or more of the following optional ingredients. The cosmetic compositions of the invention may comprise preservatives such as 2-bromo2-nitropropane-1,3-diol (bronopol, which is available commercially under the trade name Myacide RTM), benzyl alcohol, diazolidinyl urea, imidazolidinyl urea, methyl paraben, phenoxy ethanol, ethyl paraben, propyl paraben, sodium methyl paraben, sodium dehydroacetate, polyhexamethylenebiguanide hydrochloride, sodium benzoate, chlorhexidine digluconate, isothiazolone and sodium propyl paraben, suitably in an amount of from 0.01% to 10% by weight of the cosmetic composition.

The cosmetic compositions of the invention may comprise thickeners, viscosity modifying agents and/or gelling agents, such as acrylic acid polymers e.g. available commercially under the trade name Carbopol, Ultrez or Novethix (Lubrizol) or Sepigel, Sepiplus and Simulgel (Seppic). Modified celluloses may also be added, for example hydroxyethylcellulose available commercially under the trade name Natrosol (Hercules) or hydroxypropylmethyl cellulose, amine oxides, block polymers of ethylene oxide and propylene oxide (for example, those available from BASF Wyandotte under the trade name "Pluronic"RTM), PVM, MA, or a decadiene crosspolymer (available under the trade name Stabilez 60), ethoxylated fatty alcohols, salt (magnesium chloride, sodium chloride), Aristoflex AVC (Clariant), phthalic acid amide, xanthan gum, sodium polyacrylate, polyvinyl alcohols, fatty alcohols and alkyl galactomannans available under the trade name N-Hance from Hercules, suitably in an amount of from 0.1% to 10% by weight of the cosmetic composition.

Sequestering agents may be added to the cosmetic compositions, such as ethylenediamine tetraacetic acid and salts thereof, suitably in an amount of from 0.005% to 0.5% by weight of the cosmetic composition.

The cosmetic compositions may also include waxes such as cocoa butter suitably in an amount of from 1% to 99% by weight of the cosmetic composition.

The cosmetic compositions may also comprise suitable, cosmetically acceptable diluents, carriers and/or propellants such as dimethyl ether.

The cosmetic compositions may also include pearlising agents such as stearic monoethanolamide and/or mica, suitably in an amount of from 0.01% to 10% by weight of the cosmetic composition.

The cosmetic compositions may include perfumes. In some embodiments the perfumes are present in an amount of from 0.01% to 2% by weight of the cosmetic compositions, as may water soluble dyes such as tartrazine, suitably in an amount of from a trace amount (such as 1 x 10-5 %) to 0.1 % by weight of the cosmetic composition.

The cosmetic compositions may also include pH adjusting agents such as sodium hydroxide, aminomethyl propanol, triethanolamine, suitably in an amount of from 0.01 % to 10% by weight of the cosmetic composition. The cosmetic compositions may be buffered by means well known in the art, for example by use of buffer systems comprising succinic acid, citric acid, lactic acid, and acceptable salts thereof, phosphoric acid, mono-or disodium phosphate and sodium carbonate. Suitably, the cosmetic composition may have a pH between 3 and 10, preferably between 4 and 8.

### Kit

In further embodiments, the kit of the fourth aspect may also comprise a set of instructions. In some embodiments the instructions direct the user to apply the first and second cosmetic compositions to the skin, for example, the skin of the face, neck and/or décolleté. In some embodiments the instructions direct the user to apply the first cosmetic composition before the second composition. For example, the instructions may direct the user to apply the first cosmetic composition at least 5 minutes before, at least 2 hours before, at least 4 hours, before, at least 6 hours before, at least 8 hours before, or at least 10 hours before the second cosmetic composition. In Some embodiments, the instructions direct the user to apply the cosmetic compositions of the kit of the fourth embodiment in accordance with the method of the fifth embodiment.

### Methods of Use

The invention relates in a third aspect to a method for stimulating the production of dermal extracellular proteins including collagen, fibrillin, fibronectin and decorin in humans with the cosmetic composition of the invention. The method comprises topically applying the cosmetic compositions of the first or second aspects.

The invention relates in a fifth aspect to a method for stimulating the production of dermal extracellular proteins including collagen, fibrillin, fibronectin and decorin in humans with the kit of the invention. The method of the invention comprises the application of the first cosmetic composition and second cosmetic composition of the kit of the fourth aspect to the skin of said human. In some embodiments, the second cosmetic composition is applied after the first cosmetic composition.

In some embodiments, the method of the fifth aspect comprises a delay between the application of the two cosmetic compositions. For example, in some embodiments of the method the first composition is applied at least 2 hours before the second composition, for example, at least 4 hours, at least 6 hours, at least 8 hours or at least 10 hours before the second composition. In some embodiments the method requires that the second composition is applied no more than 12 hours after the second composition, for example, no more than 10 hours, no more than 8 hours, no more than 6 hours or no more than 4 hours. In a further embodiment, the second composition should be applied within a certain time period following the application of the first composition. For example, the second composition should be applied between 2 and 12 hours after the first composition, e.g., between 2 and 8 hours, between 4 and 10 hours, between 6 and 12 hours, or between 6 and 10 hours. In a preferred embodiment, the second composition should be applied between 8 and 10 hours after the first composition.

In embodiments of the method of either the third or fifth aspect, the method further comprises topically administering the cosmetic composition or compositions of the kit to the face, neck and décolleté skin of said human. In a most preferred embodiment, the method comprises topically administering the cosmetic composition or compositions of the kit to the skin of the face of said human.

### Use

The invention also relates in a sixth aspect to the use of the cosmetic composition comprising oligo-alpha-glucans and a tetrapeptide of the first or second aspect, or the kit of the fourth aspect, as a non-therapeutic cosmetic treatment to improve the condition of the skin and/or lines and/or wrinkles and/or imperfections.

### Examples

The invention is further described by the following examples.

### Experimental Procedure

The activities of the skin care ingredients of the invention were assessed using two different methods A) enzyme-linked immunosorbent assay (ELISA) and 2) Immunofluorescence. An ELISA is an immunological assay commonly used to measure presence and abundance of specific proteins (antibodies, antigens, proteins and glycoproteins) in biological samples.

ELISA was used to quantify the expression of the newly synthesized extracellular protein collagen-1 in the culture supernatant medium. The quantity present was evaluated by how much binds to immobilized antibodies on the ELISA plate, which specifically recognize the carboxy-terminal pro-peptide of procollagen type I. This binding of pro-collagen to antibody is then visualised first by the addition of an enzyme-linked secondary antibody. A substrate is then added to induce a colour change proportional to the amount of bound procollagen type I.

Immunofluorescence relies on the use of antibodies to label a specific protein with a fluorescent dye such as fluorescein isothiocyanate (FITC). In the following experiments, indirect immunofluorescence was used. indirect immunofluorescence is where a primary antibody first recognises and attaches to the specific protein, in this case fibrillin-1, and then a secondary antibody conjugated with FITC directed against the primary antibody is used for detection.

For all experiments, the control liquid stock was prepared by dissolving the powdered peptides in dimethyl sulfoxide (DMSO). Ingredients were dissolved in DMSO before further dilution in cell culture medium and combined to obtain the desired ratios.

For the collagen-1 ELISA assay, Normal Human Fibroblast cells (NHFs) were cultured in contact with the cosmetic ingredients of the invention, or composition without the ingredients (negative control), for 72 hours. Thereafter the cultured supernatants were removed, and the quantity of collagen-1 determined using an ELISA assay for the protein of interest. For the ELISA quantification, cell viability was first calculated using Hoechst staining allowing the quantity of collagen-1 to be weighted to the number of viable cells.

Quantification of fibrillin-1 was performed using immunofluorescence. Primary Human Dermal Fibroblast (HDF) cells were purchased from Promo Cell (Heidelberg, Germany). Cells were grown in the wells of 96-well black walled, clear bottomed, plates (Pierce, Fisher Scientific, Loughborough, UK). Cells were cultured in Human Dermal Fibroblast media supplemented with Basic Fibroblast Growth Factor (recombinant human), 1 ng/ml and Insulin 5 µg/ml. Cells were cultured for 5 days in the presence of the active ingredients either on their own or in combination, changing media and ingredients every 48 hours, followed by immunofluorescence assessment. At the time of treatment cells were at passage 3 - 4. After 5 days the media was discarded, and cells fixed with methanol. Fibrillin-1 fibres were probed using primary Fibrillin-1 monoclonal antibody (11C1.3; mouse monoclonal antibody (Thermo Fisher, Fisher Scientific, Loughborough, UK) 1 in 100 dilution overnight). Secondary Goat anti-mouse IgG (Thermo Fisher; 1 part antibody in 1000 buffer solution for 1 hour) was then added, which includes a fluorescent tag and binds to the primary antibody. The antibody binding to Fibrillin-1 fibres was visualised using a Nikon ECLIPSE Ti2-E microscope (Nikon, Tokyo, Japan) using an excitation lens for fluorescein isothiocyanate (FITC). Fibrillin-1 fibre abundance was quantified by setting an intensity threshold and calculating the percentage coverage at or above that threshold in each image.

Results are expressed as an average percentage increase in fibrillin abundance compared to untreated control.

### Example Formulations

The following are examples of cosmetic compositions or kits according to the invention. They are provided as exemplary compositions only and are not intended to be limiting on the invention.

**Cosmetic Composition 1 - Representative water-in-oil emulsion cosmetic composition**

| Material | % w/w |
|---|---|
| Dimethicone | 13.83 |
| Water | 38.11 |
| Glycerin | 5.00 |
| Dimethicone crosspolymer & Dimethcone | 32.31 |
| Butylene glycol | 2.60 |
| PEG/PPG-18/18 dimethicone & Polyglyceryl -4 isostearate & Hexyl laurate | 3.00 |
| Cetyl PEG/PPG-10/1 dimethicone | 2.00 |
| Magnesium sulphate | 0.60 |
| Phenoxyethanol & Methylparaben & Ethylparaben | 0.55 |
| propanediol & pentylene glycol & decyl glucoside & water | 1.986 |
| Oligo-alpha- glucans | 0.01 |
| Pal-LSPD-OH | 0.004 |

### Method of manufacture

1. In the main vessel add Dimethicone, Dimethicone crosspolymer, PEG/PPG 18/18 dimethicone & polyglyceryl-4 isostearate & hexyl laurate and Cetyl PEG/PPG-10/1 dimethicone to make the oil phase.
2. Separately weigh out water, magnesium sulphate, glycerine, phenoxyethanol & methylparaben & ethylparaben and peptide & propanediol & pentylene glycol & decyl glucoside, & water and oligo-alpha-glucans, stir until solids are dissolved to make the water phase.

Add the water phase to the oil phase slowly with constant stirring at high speed (creating a vortex). Continue stirring for 5 minutes.

Homogenise the product for 5 minutes at 3500 rpm using a Silverson mixer or equivalent.

**Cosmetic Composition 2 - Representative oil-in-water emulsion cosmetic composition**

| Material | % w/w |
|---|---|
| Dimethicone | 5.50 |
| Water | 79.95 |
| Glycerin | 5.00 |
| Dimethicone crosspolymer & Dimethicone | 1.00 |
| Phenoxyethanol & Methylparaben & Ethylparaben | 0.80 |
| Glyceryl stearate & PEG-100 stearate | 2.00 |
| Cetearyl alcohol | 2.00 |
| Sodium polyacrylate | 0.60 |
| Xanthan gum | 0.10 |
| Tetrasodium EDTA | 0.05 |
| propanediol & pentylene glycol & decyl glucoside & water | 1.986 |
| Oligo-alpha- glucans | 0.01 |
| Pal-LSPD-OH | 0.004 |

### Method of manufacture

To water add glycerine and dissolve tetrasodium EDTA.
Using homogenisation sprinkle in xanthan gum and continue to homogenise for 5 minutes or until hydrated.
Heat water phase to 70-75°C.
In a separate vessel weigh out oil phase and heat to 70-75°C. (Dimethicone, cetearyl alcohol, glyceryl stearate & PEG-100 stearate)
When at temperature stir in the sodium polyacrylate.
With both phases at 70-75°C add the oil phase to the water phase and homogenise for 2 minutes.
Cool to room temperature.
Add dimethicone crosspolymer & dimethicone and homogenise for 2 minutes.
Stir in phenoxyethanol & methylparaben & ethylparaben and peptide & propanediol & pentylene glycol & decyl glucoside & water.
Make to weight with water and stir smooth.

**Cosmetic Composition 3 - Representative Gel-based cosmetic composition**

| Material | % w/w |
|---|---|
| Glycerin | 5.00 |
| Propanediol | 2.00 |
| Acrylates/C10-30 alkyl acrylate crosspolymer | 1.00 |
| Alcohol denat. | 0.50 |
| Phenoxyethanol & Methylparaben & Ethylparaben | 0.40 |
| Potassium hydroxide | 0.29 |
| Tetrasodium EDTA | 0.05 |
| propanediol & pentylene glycol & decyl glucoside & water | 1.984 |
| Oligo-alpha- glucans | 0.01 |
| Pal-LSPG-OH | 0.004 |
| Myr-GPKG-OH | 0.002 |
| Water | 87.76 |

### Method of manufacture

1. To water add glycerine and propanediol and dissolve tetrasodium EDTA.
2. Using homogenisation sprinkle in acrylates/C10-30 alkyl acrylate crosspolymer and continue to homogenise for 5 minutes or until hydrated.

Stir in potassium hydroxide to form gel.

Stir in alcohol denat., phenoxyethanol & methylparaben & ethylparaben and peptide & propanediol & pentylene glycol & decyl glucoside & water.

Make to weight with water and stir smooth.

**Cosmetic Composition 4 - Representative oil-in-water emulsion cosmetic composition (most representative of Revival night cream)**

| Material | % w/w |
|---|---|
| Dimethicone | 2.00 |
| Butyrospermum parkii (Shea) butter | 3.00 |
| Water | To 100 |
| Glycerin | 7.00 |
| Polyacrylamide & C13-C14 isoparaffin & water | 3.00 |
| Phenoxyethanol & Caprylyl glycol & Ethylhexylglycerin, | 0.60 |
| Sodium benzoate & water | 2.00 |
| Caprylic/ capric triglyceride, | 4.00 |
| Sucrose cocoate & Sorbitan stearate | 2.50 |
| Cetearyl alcohol | 2.50 |
| Xanthan gum | 0.10 |
| Tetrasodium EDTA | 0.05 |
| Butylene glycol, Dipropylene glycol, propylene glycol | 1.20 |
| Oligo-alpha- glucans | 0.01 |
| Acetyl dipeptide-1 cetyl ester | 0.005 |
| Palmitoyl oligopeptide and palmitoyl tetrapeptide | 0.001 |
| Vitamins A,and E (Retinol, Tocopherol acetate) | 0.40 |
| Vitamin C (Ascorbyl glucoside) & Water | 2.00% |

### Method of manufacture

To water add glycerine, Butylene glycol, Dipropylene glycol, propylene glycol and dissolve tetrasodium EDTA.
Using homogenisation sprinkle in xanthan gum and continue to homogenise for 5 minutes or until hydrated.
Heat water phase to 75-80°C then add Sucrose cocoate & Sorbitan stearate and stir until dissolved
In a separate vessel weigh out oil phase and heat to 70-75°C. (Dimethicone, cetearyl alcohol, Butyrospermum parkii (Shea) butter, Caprylic/ capric triglyceride, Vitamins A & E).
With both phases at 70-75°C add the oil phase to the water phase and homogenise for 2 minutes.
Cool to room temperature.

### Kit 1 - Representative oil-in-water emulsion first and second compositions

**First Composition:**

| Material | % w/w |
|---|---|
| Dimethicone | 5.50 |
| Water | 79.95 |
| Glycerin | 5.00 |
| Dimethicone crosspolymer & Dimethicone | 1.00 |
| Phenoxyethanol & Methylparaben & Ethylparaben | 0.80 |
| Glyceryl stearate & PEG-100 stearate | 2.00 |
| Cetearyl alcohol | 2.00 |
| Sodium polyacrylate | 0.60 |
| Xanthan gum | 0.10 |
| Tetrasodium EDTA | 0.05 |
| propanediol & pentylene glycol & decyl glucoside | 1.99 |
| Oligo-alpha- glucans | 0.01 |

**Second Composition:**

| Material | % w/w |
|---|---|
| Dimethicone | 5.50 |
| Water | 79.95 |
| Glycerin | 5.00 |
| Dimethicone crosspolymer & Dimethicone | 1.00 |
| Phenoxyethanol & Methylparaben & Ethylparaben | 0.80 |
| Glyceryl stearate & PEG-100 stearate | 2.00 |
| Cetearyl alcohol | 2.00 |
| Sodium polyacrylate | 0.60 |
| Xanthan gum | 0.10 |
| Tetrasodium EDTA | 0.05 |
| propanediol & pentylene glycol & decyl glucoside | 1.996 |
| Pal-LSPD-OH | 0.004 |

*Method of manufacture*

## Claims

1. A cosmetic composition comprising:
oligo-alpha-glucans; and
a tetrapeptide
wherein the tetrapeptide is selected from the group consisting of;
a) tetrapeptides having the amino acid sequence U-LSXX-Z wherein L is used to denote amino acid Leucine and S is used to denote Serine, as per the internationally recognised single letter code for amino acids and X denotes an amino acid selected from the group consisting of Valine (V), Aspartic acid (D), Proline (P), Glycine (G) and mixtures thereof,
b) tetrapeptides having the amino acid sequence U-GPXG-Z wherein G is used to denote amino acid glycine and P denotes the amino acid proline, as per the internationally recognised single letter code for amino acids, X denotes an amino acid independently selected from the group consisting of Lysine (K), Glutamic acid (E) and Serine (S) and mixtures thereof,
c) tetrapeptides having the amino acid sequence U-XXGD-Z wherein G is used to denote amino acid Glycine and D is used to denote amino acid Aspartic acid, as per the internationally recognised single letter code for amino acids and X denotes an amino acid selected from the group consisting of Glutamic acid (E), Lysine (K), Leucine (L), Alanine (A), Isoleucine (I), Arginine (R) and mixtures thereof,
d) tetrapeptides having the amino acid sequence U-QTAV-Z wherein Q is used to denote amino acid Glutamine, T is used to denote amino acid Threonine, A is used to denote amino acid Alanine and V is used to denote amino acid Valine as per the internationally recognised single letter code for amino acids, and
e) combinations thereof
wherein at the N-terminal end of the one or more tetrapeptide, U is selected from the group consisting of H, -CO-R¹, -SO₂-R¹ or a biotinyl group,
wherein at the C-terminal end, Z is selected from the group consisting of OH, OR¹, NHR¹ or NR¹R², and
wherein R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N.

2. A kit comprising:
a first cosmetic composition comprising oligo-alpha-glucans; and
a second cosmetic composition comprising a tetrapeptide,
wherein the tetrapeptide is selected from the group consisting of;
a) tetrapeptides having the amino acid sequence U-LSXX-Z wherein L is used to denote amino acid Leucine and S is used to denote Serine, as per the internationally recognised single letter code for amino acids and X denotes an amino acid selected from the group consisting of Valine (V), Aspartic acid (D), Proline (P), Glycine (G) and mixtures thereof,
b) tetrapeptides having the amino acid sequence U-GPXG-Z wherein G is used to denote amino acid glycine and P denotes the amino acid proline, as per the internationally recognised single letter code for amino acids, X denotes an amino acid independently selected from the group consisting of Lysine (K), Glutamic acid (E) and Serine (S) and mixtures thereof,
c) tetrapeptides having the amino acid sequence U-XXGD-Z wherein G is used to denote amino acid Glycine and D is used to denote amino acid Aspartic acid, as per the internationally recognised single letter code for amino acids and X denotes an amino acid selected from the group consisting of Glutamic acid (E), Lysine (K), Leucine (L), Alanine (A), Isoleucine (I), Arginine (R) and mixtures thereof,
d) tetrapeptides having the amino acid sequence U-QTAV-Z wherein Q is used to denote amino acid Glutamine, T is used to denote amino acid Threonine, A is used to denote amino acid Alanine and V is used to denote amino acid Valine,
e) tetrapeptides having the amino acid sequence RSRK or U-GQPR, most preferably N-palmitoyl-GQPR, wherein R is used to denote amino acid Arginine, S is used to denote amino acid Serine, K is used to denote amino acid Lysine, G is used to denote amino acid Glycine, Q is used to denote amino acid Glutamine, and P is used to denote the amino acid proline as per the internationally recognised single letter code for amino acids, and
f) combinations thereof
wherein at the N-terminal end of the one or more tetrapeptide, U is selected from the group consisting of H, -CO-R¹, -SO₂-R¹ or a biotinyl group,
wherein at the C-terminal end, Z is selected from the group consisting of OH, OR¹, NHR¹ or NR¹R², and
wherein R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N.

3. The cosmetic composition of claims 1 or the kit of claim 2 wherein the oligo-alpha glucans are provided in the form of an extract from the stems, roots, leaves, seeds and/or fruit of a plant.

4. The cosmetic composition or kit of claim 3, wherein the oligo-alpha-glucans are provided in the form of an extract from the roots of lindera strychnifolia, wherein, optionally the extract is subjected to an enzymatic hydrolysis step.

5. A cosmetic composition comprising:
oligo-alpha-glucans, provided in the form of an extract from the roots of lindera strychnifolia, wherein, optionally the extract is subjected to an enzymatic hydrolysis step; and
a tetrapeptide
wherein the tetrapeptide is selected from the group consisting of;
tetrapeptides having the amino acid sequence RSRK and U-GQPR, most preferably N-palmitoyl-GQPR, wherein R is used to denote amino acid Arginine, S is used to denote amino acid Serine, K is used to denote amino acid Lysine, G is used to denote amino acid Glycine, Q is used to denote amino acid Glutamine, and P is used to denote the amino acid proline as per the internationally recognised single letter code for amino acids,
wherein at the N-terminal end of the one or more tetrapeptide, U is selected from the group consisting of H, -CO-R¹, -SO₂-R¹ or a biotinyl group,
wherein R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N.

6. The cosmetic composition of claim 1 or claims 3 to 5, or the kit of claims 2 to 4, wherein the oligo-alpha-glucans have a degree of polymerisation of from 2 to 10, optionally from 2 to 6.

7. The cosmetic composition or kit of claim 6 wherein at least 80% of the oligo-alpha-glucans consist of 2 to 6 glucose monomers.

8. The cosmetic composition of claim 1 or claims 3 to 7 or the kit of claims 2 to 4 or claims 6 to 7, wherein tetrapeptide is provided in a controlled release form.

9. The cosmetic composition of claim 1 or claims 3 to 8 or the kit of claims 2 to 4 or claims 6 to 8, wherein the oligo-alpha-glucans are present in an amount of 0.00001 wt% to 5 wt% of the total composition, preferably 0.0001 wt% to 2 wt% of the total cosmetic composition, most preferably 0.0001% to 0.5 wt% of the total cosmetic composition.

10. The cosmetic composition of claim 1 or claims 3 to 9 or the kit of claims 2 to 4 or claims 6 to 9, wherein the tetrapeptide is present at from 0.00001 wt% to 2 wt% of the total cosmetic composition, preferably 0.0001 wt% to 1 wt% of the total cosmetic composition, most preferably 0.0005 wt% to 0.1 wt% of the total cosmetic composition.

11. The cosmetic composition of claim 1 or claims 3 to 10 or the kit of claims 2 to 4 or claims 6 to 10 wherein:
a) U of the tetrapeptide is independently selected from the group consisting of octanoyl (C8), decanoyl (C10), lauroyl (C12), myristoyl (C14), palmitoyl (C16), stearoyl (C18), biotinoyl, elaidoyl, oleoyle and lipoyle, and/or
b) Z of the tetrapeptide is selected from the group consisting of OH, methoxy, ethoxy and NH₂, preferably OH.

12. The cosmetic composition of claim 1 or claims 3 to 11, or the kit of claims 2 to 4 or 6 to 11, wherein the cosmetic composition comprising the tetrapeptide further comprises a matrix metalloproteinase inhibitor, wherein the matrix metalloproteinase inhibitor is optionally selected from the group consisting of retinoid, N-acetyl cysteine, glutathione, 2-furildioxime, vitamin C, flavones, isoflavones, hydrolysed rice protein, alfalfa extract, white lupin, zizyphus jujube extract, dihydroxy methyl chromone, kudzu extract, vitis vinifera extract, oenothera biennis extract, anogeissus leiocarpus extract, soyabean extract, phyllanthus emblica extract, lentinus edodes extract, argania spinosa extract, paeonia lactiflora extract, pisum sativum extract, cistus monspellensis extract, litchi chinensis pericarp extract, plantago lanceolata extract, salicyloyl phytosphingosine, cyanidium caldarium extract, lactobionic acid, stevia rebaudiana extract, acetyl tripeptide-30 citrulline, morus alba extract, and mixtures thereof.

13. A method for stimulating the production of dermal extracellular proteins in humans, the method comprising administering to the skin of said human a cosmetically effective amount of a cosmetic composition according to claim 1 or claims 3 to 12.

14. A method for stimulating the production of dermal extracellular proteins in humans, the method comprising administering to the skin of said human a cosmetically effective amount of the first and second cosmetic compositions of the kit of claims 2 to 4 or claims 6 to 12 wherein, optionally, the first cosmetic composition is applied;
a) at least 2 hours before the second cosmetic composition;
b) at least 4 hours before the second cosmetic composition; or
c) at least 8 hours before the second cosmetic composition.

15. Use of a cosmetic composition according to any of claims 1 or claims 3 to 12 or the kit according to any one of claims 2 to 4 or claims 6 to 12 for the non-therapeutic cosmetic treatment to improve the condition of the skin and/or lines and/or wrinkles and/or imperfections.
